# EUROPEAN PATENT SPECIFICATION

(11) **EP 1 231 962 B1**
(45) Date of publication and mention of the grant of the patent: **10.11.2004**
(21) Application number: 00977699.8
(22) Date of filing: 23.11.2000
(51) Int. Cl.: A61M 5/32, A61M 5/50

(54) **SYRINGE WITH RETRACTABLE NEEDLE**
SPRITZE MIT EINZIEHBARER NADEL
Seringue avec aiguille retractable

(30) Priority: 24.11.1999 GB 9927713
(43) Date of publication of application: 21.08.2002
(73) Proprietor: SAFE-T-LIMITED, Lonan, Isle of Man IM4 7JD (GB)
(72) Inventor: JEFFREY, Peter, Liverpool L19 9DH (GB)
(74) Representative: Barker, Rosemary Anne
(86) International application number: PCT/GB2000/004457
(87) International publication number: WO 2001/037902

(56) References cited:
- WO-A-92/18187
- AT-B- 395 679
- US-A- 5 578 011
- US-A- 5 993 417

## Description

### FIELD OF INVENTION

This invention relates to hollow-needle devices such as hypodermic syringes and body fluid samplers; and has particular relevance to such devices with provision for automatic full retraction of the hollow-needle after use for device contents expression, usually after a single such use.

### BACKGROUND TO INVENTION

WO-A-92/18187 concerns such devices where needle retraction involves entry of a needle-carrying hub into hollow interior of a plunger capable of serving for device contents expression through the needle. During such contents expression, of course, the plunger needs its end later to receive the needle-carrying hub to be closed off and in sealed relation to the contents being expressed, but in a manner that can and will reliably be so disrupted after contents expression as to permit desired retraction of needle and hub parts into the hollow interior of the plunger. A disruptable closure, such as a piercable or frangible membrane as a seal across the end of the plunger is technically feasible, including as to being disrupted as required at and for needle retraction, typically by sharp projections from the needle-carrying hub itself. However, difficulties can arise in terms of achieving all desiderata, particularly for an intendedly single-use, i.e. disposable, hollow-needle device.

Specifically, for what must be a high volume product made using high speed moulding and automatic assembly of its parts at low finished cost, there are problems for consistently and reliably achieving required full sealing indefinitely and full disruption always allowing required free passage of parts. These requirements become yet more difficult to meet when allied with seeking to provide that fully effective disruption of the end closure of the plunger be achieved with no or low preferably un-noticably insignificant increase in required force applied to the plunger compared with that used for contents expression. A displacable sliding plug will normally fail at least in such respect.

It is an object of this invention to provide plunger closure means with better capability to meet desiderata for an automatically retractable hollow-needle device.

### SUMMARY OF INVENTION

According to the invention as defined in claim 1, there is provided a hollow needle device comprising a hollow plunger, a body part affording a cylindrical chamber within which the plunger is sealingly slidable, a needle-carrying hub for automatic retraction into the plunger, and a closure for the plunger, the closure comprising a member normally in sealing relation with end-adjacent internal provision of the hollow plunger but disengageable therefrom and bodily movable into the hollow plunger by localised first engagement of the member at or near end of contents expression stroke of the plunger. The localised first engagement is asymmetric and causes tipping of the closure member.

US Patent No 5,578,011 has been brought to our attention as prior art showing localised displacing engagement of a syringe plunger closure member, but without asymmetry of such engagement and with only axial displacing movement of the closure member. According to preferred embodiments of the invention, the sealing relation of the closure member may be peripheral of the closure member and the localised displacing engagement may be eccentric within such periphery, at least for a stiff said closure member that is substantially undeformed in being dislodged in a manner involving tipping first movement.

The needle-carrying hub may itself make direct displacing engagement with the closure member and either or both of the hub and the member may have provision to assure asymmetry of that engagement at the end of said contents-expression stroke of the plunger in the body part.

For a typical hollow-needle syringe-type device of substantially circular cylindrical structure, the closure member can be a flat disc of less diameter than interior of the plunger and having a peripheral groove within thickness of its edge to fit to an internal circumferential rib adjacent the mouth of the plunger. Alternatives for the closure member include anything other than flat that nonetheless presents an annular groove to fit to said rib and otherwise permits easy clearance passage of the closure member along interior of the plunger away from its mouth.

Typical engagement provisions for the needle-carrying hub and the closure member include a localised protrusion from the hub at a position eccentric of the closure member, say as a peg or tooth provision that may be of integrally moulded nature. The opposite is equally feasible, i.e. localised eccentric protrusion from the closure member, or some combination; as is use of any desired intermediate member carrying either or both such localised protrusion(s).

It will be recognised that above substantially flat edge-grooved closure member is a particular application of so-called "tippable discs", a class of devices long known as capable when suitably edge-supported of resisting substantially uniformly applied force much greater than an asymetrically applied tipping displacement force sufficient to disturb the disc from its edge-support. Given that syringe-type devices hereof normally express contents of liquid form, thus inevitably involve uniform fluid pressures, there is a near-ideal context for application of tippable discs. However, there is well over a decade of highly numerous proposals for automatic needle retraction in syringe-type devices and none to date have made this connection and so-applied tippable discs. The force discrepancy, namely much lower for dislodgement than is readily withstood applied evenly, is of profound significance to syringe-type hollow-needle devices intendedly for one use only. Human tactile capabilities are extremely sensitive, and trainable by experience. Almost any increase in force required to achieve retraction would become detectable, even if very slight, and so facilitate defeating the objective of limitation to single use; but such action actually well below necessarily applied contents expression forces will not detectable in that way.

Moreover, the force discrepancy affords a margin of manufacturing tolerance without risk of failure to retraction operation that is exceptionally valuable for making low cost devices and parts thereof.

### BRIEF DESCRIPTION OF DRAWINGS

Specific exemplary implementation for this invention is now described and shown in the accompanying drawing, in which
Figure 1 is a longitudinal axial sectional view through a syringe-type hollow-needle device 100 with plunger end-sealing as taught herein;
Figure 2 is a cross section at X-X of Figure 1;
Figure 3 is an exploded scrap sectional view showing detail of parts involved in plunger end sealing and unsealing; and
Figures 4A, B and C are similar views showing such parts during contents expression, approaching end thereof, and during automatic needle retraction, respectively.

### ILLUSTRATED EMBODIMENT

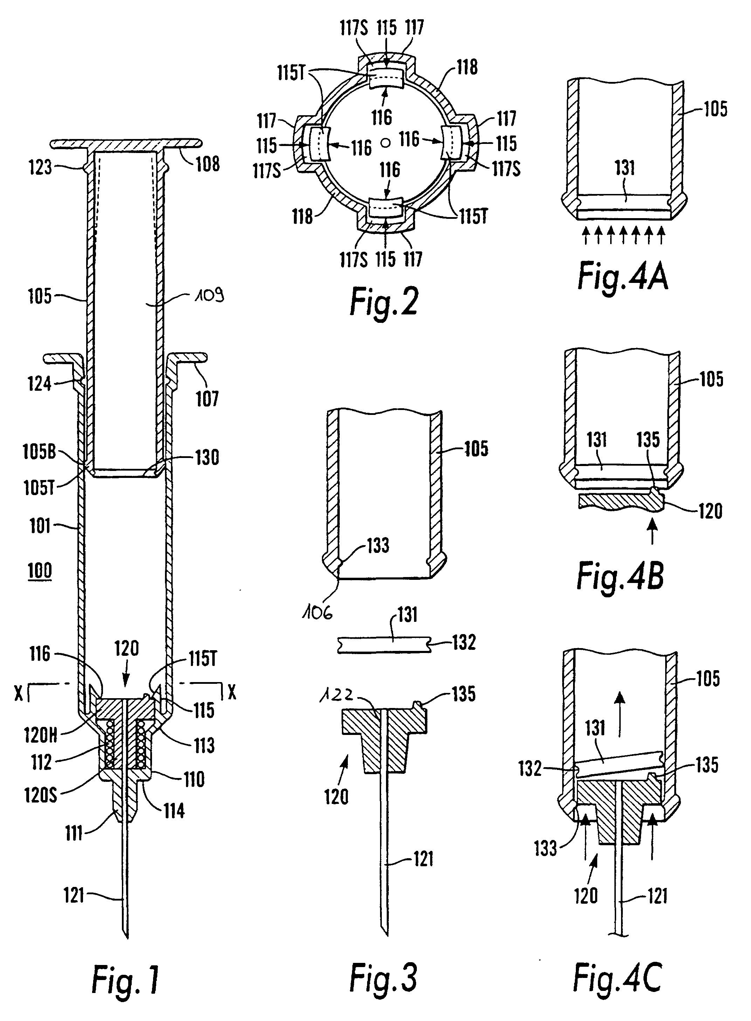

Referring first to Figures 1 and 2, the hollow-needle syringe-type device 100 has an advantageously one-piece moulded outer body part of elongate hollow conveniently circular cylindrical form that presents a main chamber 101 within which a hollow plunger 105 is sealingly slidable, a forward or extension chamber 110 to a hollow-needle exit nose 111 and housing a retraction spring 112; and internal deflectable arm-type latching formations 115 to hold a needle-carrying hub 120 until released by deflection for needle retraction when engaged by end 106 of the plunger 105. The device 100 is generally as disclosed in published PCT patent specification No WO 92/18187, including convenient outer body part steppings shown at 113 from the main chamber 101 to the forward or extension chamber 110 and at 114 from the latter to the nose 111, and end grip flanging 107 to cooperate with end flanging 108 of the plunger 105.

Inner end formation of the plunger 105 includes outer peripheral bead 105B that can usefully make a direct sliding seal in compressive engagement by interior of the main chamber 101, and is end-tapered at 105T to cooperate with end-tapering 115T to inward latching teeth 116 at ends of the deflectable arms 115. The arms 115 are shown extending from tapered stepping 113 in localised accommodations 117 between reductions 118 of the outer body part, and the teeth 116 capture head 120H of top-hat or T-section needle-carrying hub 120 with retraction spring 112 held retracted about stem 120S of the hub 120. Engagement of the end-tapering 105T of the plunger 105 with end-tapering 115T of the arms 115 deflects the arms 115 into sufficient spacing 117S in accomodations 117 to release the hub 120 for retraction forced by the spring 112 through end seal 130 of the hollow plunger 105 into its interior 109 complete with needle 121 in fixed relation with through-bore 122 of the hub 120. The plunger 105 is preferably latched when fully entrant the outer body part, see cooperating rib and groove formations 123, 124.

The plunger end seal 130 is of tippable disc type, see further in Figures 3 and 4. Specifically, as shown, a circular disc 131 has a peripheral edge groove 132 within its thickness, and the hollow plunger 105 has a cooperating inner end-adjacent rib 133. This rib 133 is of readily bump-off nature thus not adversely affecting one-piece mouldability of the plunger 105. The disc 131 is of slighly less overall diameter than interior 109 of the hollow plunger 105 to move freely in such interior 109 as and when required. Snap-action insertion of the disc 131 into all-round engagement of its groove 132 on the plunger rib 133 is readily achieved in high-speed assembly equipment.

Liquid-tight sealing between the disc 131 and the plunger 105 is aided by assuring that mould tooling for the disc 131 has a split line close to a main face of the disc 131 rather than in the groove 132, preferably substantially clear of such groove; and by some degree of compression of the plunger 105 by walling of the main chamber 101 of the outer body part. Suitable materials for the outer body part, the plunger 105 and the disc 131 are stiff nucleated hompolymer polyethylene, less stiff high density polyethylene, and lower density polyolefin typically polypropylene or polyethylene, respectively.

The disc 131 as peripherally located and held by its groove 132 cooperating with the plunger rib 133 will resist much larger evenly applied force (as by fluid pressure as the plunger 105 expresses contents of the main chamber 101 through the needle 121) than an eccentrically applied force sufficient to dislodge the disc 131 from such location. Accordingly, head 120H of the needle-carrying hub 120 has a protruding peg formation 135 near its edge to engage the disc 131 asymmetrically and dislodge it into the interior 109 of the plunger 105 at the end of a contents expression stroke of the plunger 105.

## Claims

1. Hollow-needle device (100) comprising a hollow plunger (105), a body part (101) affording a cylindrical chamber within which the plunger (105) is sealingly slidable, a needle-carrying hub (120) for automatic retraction into the plunger (105), and a closure (130) for the plunger (105), the closure (130) comprising a member (131) normally in sealing relation with end-adjacent internal provision (133) of the hollow plunger (105) but disengageable therefrom and bodily movable into the hollow plunger (105) by localised first engagement (135) of the member (131) at or near end of contents expression stroke of the plunger (105), **characterised in that** the localised first engagement (135) is asymmetric and causes tipping of the closure member (131).

2. Hollow-needle device according to claim 1, wherein the closure member (131) has peripheral said sealing relation (132/133).

3. Hollow-needle device according to claim 2, wherein said sealing relation is aided by the plunger (105) being to some degree compressed by the body part (101).

4. Hollow-needle device according to claim 2 or claim 3, wherein the closure member (131) has said localised displacing engagement eccentric within its periphery.

5. Hollow-needle device according to claim 2, 3 or 4, wherein the closure member (131) is stiff and substantially undeformed in being dislodged in a manner involving tipping first movement.

6. Hollow-needle device according to any preceding claim, wherein the closure member (131) presents an annular groove (132) to fit to an internal circumferential rib (133) adjacent the mouth of the plunger (105), and is otherwise an easy clearance passage along interior of the plunger (105) away from its mouth.

7. Hollow-needle device according to claim 6, wherein the closure member (131) is a moulding and has a mould parts split line substantially clear of its said annular groove (132).

8. Hollow-needle device according to claim 6 or claim 7, wherein the closure member is a flat disc (131) of less diameter than interior of the plunger (105) beyond said rib (133).

9. Hollow-needle device according to any preceding claim, wherein the needle-carrying hub (120) itself serves to make direct displacing engagement (135) with the closure member (131).

10. Hollow-needle device according to claim 9, wherein the the hub (120) and/or the closure member (131) has provision (135) to assure asymmetry of said displacing engagement at the end of said contents-expression stroke of the plunger (105) in the body part (101).

11. Hollow-needle device according to claim 10, wherein said engagement provision for the needle-carrying hub (120) and the closure member (131) includes a localised protrusion (135) at a position eccentric of the closure member (131).

12. Hollow-needle device according to claim 11, wherein said localised protrusion (135) is integral with said hub (120) and/or said closure member (131).

## Patentansprüche

1. Hohlnadel-Vorrichtung (100) mit einem hohlen Kolben (105), mit einem Gehäusekörper (101), der eine zylindrische Kammer aufweist, in welcher der Kolben (105) dichtend verschiebbar ist, mit einer eine Nadel tragende Nabe (120) für einen automatischen Rückzug in den Kolben (105) und mit einem Verschluss (130) für den Kolben (105), wobei der Verschluss (130) ein Teil (131) aufweist, welches üblicherweise in einer dichten Verbindung mit einer benachbart zu dem Ende des hohlen Kolbens (105) angeordneten inneren Ausformung (133) steht, aber von dieser trennbar ist und durch einen örtlichen ersten Eingriff (135) des Teils (131) an oder nahe bei dem Ende des eine Füllung ausdrückenden Kolbenhubs des Kolbens (105) körperlich in den hohlen Kolben (105) beweglich ist, **dadurch gekennzeichnet, dass** der örtliche erste Eingriff asymmetrisch ist und ein Verkippen des Verschlussteils (131) bewirkt.

2. Hohlnadel-Vorrichtung gemäß Anspruch 1, wobei das Verschlussteil (131) entlang des Umfangs die dichte Verbindung (132 / 133) aufweist.

3. Hohlnadel-Vorrichtung gemäß Anspruch 2, wobei die dichte Verbindung dadurch unterstützt wird, dass der Kolben (105) bis zu einem bestimmten Grad durch den Gehäusekörper (101) zusammengedrückt wird.

4. Hohlnadel-Vorrichtung gemäß Anspruch 2 oder 3, wobei das Verschlussteil (131) den örtlichen verlagernden Eingriff exzentrisch innerhalb seines Umfangs aufweist.

5. Hohlnadel-Vorrichtung gemäß Anspruch 2, 3 oder 4, wobei das Verschlussteil (131) steif und im Wesentlichen undeformierbar während eines Verlagerungsvorgangs ist, was zu einer verkippenden Anfangsbewegung führt.

6. Hohlnadel-Vorrichtung gemäß einem der voranstehenden Ansprüche, wobei das Verschlussteil (131) eine ringförmige Nut (132) aufweist, die an eine innere entlang des Umfangs benachbart zu der Öffnung des Kolbens (105) angeordnete Rippe (133) angepasst ist und die anderenfalls eine Aussparung für einen einfachen Durchtritt entlang des Innenraums des Kolbens (105) weg von seiner Öffnung ist.

7. Hohlnadel-Vorrichtung gemäß Anspruch 6, wobei das Verschlussteil (131) ein Formteil ist und eine Formwerkzeug-Trennlinie im Wesentlichen entfernt von der ringförmigen Nut (132) ist.

8. Hohlnadel-Vorrichtung gemäß Anspruch 6 oder 7, wobei das Verschlussteil eine flache Scheibe (131) mit einem geringeren Durchmesser als das Innere des Kolbens (105) jenseits der Rippe (133) ist.

9. Hohlnadel-Vorrichtung gemäß einem der voranstehenden Ansprüche, wobei die die Nadel tragende Nabe (120) selbst dazu dient, unmittelbar einen verlagernden Eingriff (135) mit dem Verschlussteil (131) zu bilden.

10. Hohlnadel-Vorrichtung gemäß Anspruch 9, wobei die Nabe (120) und/oder das Verschlussteil (131) eine Ausformung (135) aufweist, um die Asymmetrie des verlagernden Eingriffs am Ende des eine Füllung ausdrückenden Kolbenhubs des Kolbens (105) in dem Gehäusekörper (101) zu gewährleisten.

11. Hohlnadel-Vorrichtung gemäß Anspruch 10, wobei die Ausformung des Eingriffs für die die Nadel tragende Nabe (120) und das Verschlussteil (131) einen örtlichen Vorsprung (135) an einer exzentrischen Position zu dem Verschlussteil (131) beinhaltet.

12. Hohlnadel-Vorrichtung gemäß Anspruch 11, wobei der örtliche Vorsprung (135) einteilig mit der Nabe (120) und/oder dem Verschlussteil (131) ist.

## Revendications

1. Dispositif d'aiguille creuse (100) comprenant un plongeur creux (105), une partie de corps (101) contenant une chambre cylindrique dans laquelle le plongeur (105) peut coulisser de manière étanche, un moyeu supportant l'aiguille (120) pour une rétraction automatique dans le plongeur (105), et un obturateur (130) pour le plongeur (105), l'obturateur (130) comprenant un élément (131) normalement en relation étanche avec un moyen interne adjacent à l'extrémité (133) du plongeur creux (105) mais pouvant être désengagé de celui-ci et déplaçable d'un seul tenant dans le plongeur creux (105) par une première prise localisée (135) de l'élément (131) à ou proche de l'extrémité d'une course de distribution de contenu du plongeur (105), **caractérisé en ce que** la première prise localisée (135) est asymétrique et entraîne un basculement de l'élément d'obturateur (131).

2. Dispositif d'aiguille creuse selon la revendication 1, dans lequel l'élément d'obturateur (131) comporte ladite relation d'étanchéité (132/133) sur sa périphérie.

3. Dispositif d'aiguille creuse selon la revendication 2, dans lequel ladite relation d'étanchéité est assistée par le plongeur (105) qui est comprimée dans une certaine mesure par la partie de corps (101).

4. Dispositif d'aiguille creuse selon la revendication 2 ou 3, dans lequel l'élément d'obturateur (131) comporte ladite prise localisée de déplacement excentrée à l'intérieur de sa périphérie.

5. Dispositif d'aiguille creuse selon la revendication 2, 3 ou 4, dans lequel l'élément d'obturateur (131) est rigide et sensiblement non déformé lorsqu'il est délogé dans une manière impliquant le premier mouvement de basculement.

6. Dispositif d'aiguille creuse selon l'une quelconque des revendications précédentes, dans lequel l'élément d'obturateur (131) présente une rainure annulaire (132) pour s'adapter sur une nervure circonférentielle interne (133) adjacente à l'embouchure du plongeur (105), qui est autrement un passage d'espacement aisé le long de l'intérieur du plongeur (105) en éloignement de son embouchure.

7. Dispositif d'aiguille creuse selon la revendication 6, dans lequel l'élément d'obturateur (131) est un moulage et comporte une ligne de séparation de parties de moule sensiblement espacée de ladite rainure annulaire (132).

8. Dispositif d'aiguille creuse selon la revendication 6 ou 7, dans lequel l'élément d'obturateur est un disque plat (131) de diamètre inférieur à l'intérieur du plongeur (105) au-delà de ladite nervure (133).

9. Dispositif d'aiguille creuse selon l'une quelconque des revendications précédentes, dans lequel le moyeu supportant l'aiguille (120) sert lui-même pour réaliser une prise directe de déplacement (135) avec l'élément d'obturateur (131).

10. Dispositif d'aiguille creuse selon la revendication 9, dans lequel le moyeu (120) et/ou l'élément d'obturateur (131) comporte(nt) un moyen (135) pour assurer l'asymétrie de ladite prise de déplacement à l'extrémité de ladite course de distribution de contenu du plongeur (105) dans la partie de corps (101).

11. Dispositif d'aiguille creuse selon la revendication 10, dans lequel ledit moyen de prise pour le moyeu supportant l'aiguille (120) et l'élément d'obturateur (131) comprend une projection localisée (135) à une position excentrée par rapport à l'élément d'obturateur (131).

12. Dispositif d'aiguille creuse selon la revendication 11, dans lequel ladite projection localisée (135) est monobloc avec ledit moyeu (120) et/ou ledit élément d'obturateur (131).
